# EUROPÄISCHE PATENTSCHRIFT

(11) **EP 3 494 385 B1**
(45) Veröffentlichungstag und Bekanntmachung des Hinweises auf die Patenterteilung: **30.09.2020**
(21) Anmeldenummer: 17752064.0
(22) Anmeldetag: 03.08.2017
(51) Int. Cl.: G01N 21/71, B07C 5/342, G01N 21/25, G01N 33/20, C22B 1/00

(54) **VORRICHTUNG UND VERFAHREN ZUR LEGIERUNGSANALYSE VON SCHROTTFRAGMENTEN AUS METALL**
DEVICE AND METHOD FOR ANALYSING THE ALLOY COMPOSITION OF METAL SCRAP FRAGMENTS
DISPOSITIF ET PROCÉDÉ D'ANALYSE D'ALLIAGE DE FRAGMENTS DE FERRAILLE EN MÉTAL

(30) Priorität: 04.08.2016 DE 102016114465
(43) Veröffentlichungstag der Anmeldung: 12.06.2019
(73) Patentinhaber: Hydro Aluminium Rolled Products GmbH, 41515 Grevenbroich (DE)
(72) Erfinder: GILLNER, Ronald, 53913 Swisttal (DE); BAUERSCHLAG, Nils, Robert, 52072 Aachen (DE)
(74) Vertreter: Cohausz & Florack
(86) Internationale Anmeldenummer: PCT/EP2017/069713
(87) Internationale Veröffentlichungsnummer: WO 2018/024843

(56) Entgegenhaltungen:
- EP-A1- 1 416 265
- DE-A1- 4 426 490

## Beschreibung

Die Erfindung betrifft eine Vorrichtung zur Legierungsanalyse von Schrottfragmenten aus Metall mit einer Analyseeinrichtung, die dazu eingerichtet ist, mittels einer lokalen Messung an einer Messposition auf der Oberfläche eines zu analysierenden Schrottfragments eine Zusammensetzungsinformation über die Legierungszusammensetzung des Schrottfragments zu bestimmen. Die Erfindung betrifft weiterhin ein Verfahren zur Legierungsanalyse von Schrottfragmenten aus Metall, insbesondere unter Verwendung der zuvor genannten Vorrichtung.

Im Stand der Technik erfolgt das Sortieren bzw. Recyceln von Aluminium über mehrere Verfahrensschritte. Diese umfassen in der Regel das Sammeln der unterschiedlichen Aluminiumschrotte, eine mechanische Aufbereitung der Schrotte und eine anschließende metallurgische Verwertung der Schrotte.

Für ein ressourceneffizientes Recycling sollte die mechanische Aufbereitung der Schrotte ein Aluminiumschrottprodukt erzeugen, das den qualitativen Ansprüchen des metallurgischen Verwertungsweges entspricht. Hierzu werden im Stand der Technik unterschiedliche Aufbereitungsschritte durchgeführt, die allerdings nur eine begrenzte Sortierung in Qualitäten bzw. Legierungszusammensetzungen der Schrotte erlaubt.

Die mechanische Aufbereitung erfolgt in der Regel über eine ein- oder mehrstufige Zerkleinerung der Schrotte, der sich dann diverse Sortierschritte anschließen. Die Sortierschritte können beispielsweise eine Trennung von Eisen und NE-Metallen über Magnetscheider, Windsichtung, Wirbelstromscheidung, sensorgestützte Sortierungen zum Beispiel mittels Röntgentransmission oder -fluoreszens, Induktion, laserinduzierte Plasmaspektroskopie (Laser Induced Breakdown Spectroscopy - LIBS) oder Nahinfrarotanalyse (NIR) bewirken. Die verfahrenstechnische Kombination dieser Sortierschritte erlaubt das Sortieren der Schrotte in unterschiedliche Aluminiumqualitäten, d.h. insbesondere abhängig von ihrer Legierungszusammensetzung.

Um unterschiedliche Aluminiumlegierungen legierungsspezifisch sortieren zu können, müssen die Gehalte eines oder mehrerer Legierungselemente der einzelnen Schrottfragmente bestimmt werden. Hierzu werden typischerweise Systeme für laserinduzierte Plasmaspektroskopie (LIBS) oder Röntgenfluoreszenz (XRF) eingesetzt. Die mit diesen Systemen erzeugten Analyseergebnisse werden mit vorgegebenen Legierungszusammensetzungen verglichen und die jeweiligen Schrottfragmente der passenden Legierungszusammensetzung zugeordnet. Wird beispielsweise bei der Analyse eines Schrottfragments ein Mg-Gehalt von 5% ermittelt, so wird dieses Schrottfragment beispielsweise einer Klasse "Mg5-Legierung" zugeordnet.

Ein grundsätzliches Problem bei der Analyse von Schrottfragmenten und einer darauf basierenden Sortierung der Schrottfragmente sind Oberflächenverunreinigungen der einzelnen Schrottfragmente, die die Metalloberfläche der Schrottfragmente bedecken und damit eine Analyse erschweren. Bei den Oberflächenverunreinigungen kann es sich beispielsweise um Lacke (z.B. bei Schrottfragmenten von lackierten Kraftfahrzeugkomponenten), Eloxierschichten, Kunststoffschichten (z.B. bei Schrottfragmenten von Verbundmaterialien wie kunststofflaminierten Aluminiumprodukten) oder sogenannte Querverschmutzungen aus der Produktion (z.B. Walzöle), dem Sammel- und/oder Zerkleinerungsprozess handeln, die beispielsweise infolge von Restfeuchte an den Schrottfragmenten anhaften können.

Die Oberflächenverunreinigungen entsprechen in ihrer chemischen Zusammensetzung nicht der darunter liegenden Metallmatrix, insbesondere Aluminiummatrix. Diese Oberflächenverunreinigungen haben daher Auswirkungen auf Analyse- bzw. Sortiersystem, die die oberflächennahe Stoffzusammensetzung ermitteln, da die Analyseergebnisse durch die Oberflächenverunreinigungen stark verfälscht werden. Wird die Oberflächenzusammensetzung in einem verschmutzten Oberflächenbereich eines Schrottfragments bestimmt, so ergibt die Analyse anstelle der Zusammensetzung der Legierung des Schrottfragments stattdessen die chemische Zusammensetzung der Oberflächenverunreinigung oder eine Mischung aus der Zusammensetzung der Oberflächenverunreinigung und der darunter liegenden Metallmatrix. Die Analyseergebnisse sind daher nicht eindeutig bzw. stark fehlerbehaftet.

Zwar werden bei der lasergestützten Legierungsanalyse, beispielsweise LIBS, in der Regel mehrere Laserschüsse auf eine Stelle eines Schrottfragments aufgebracht, so dass eine mögliche Oberflächenverunreinigung teilweise abgetragen (ablatiert) wird. Es ist jedoch nicht gewährleistet, dass Oberflächenverunreinigungen an der Messposition vollständig verdampfen und die reine Metallmatrix freilegen, so dass die Analyseergebnisse weiterhin stark fehlerbehaftet sein können.

Die DE 44 26 490 A1 offenbart eine Vorrichtung und ein Verfahren zur Analyse von metallischen Teilen mittels Laserlicht.

Die EP 1 416 265 A1 offenbart ein Abtastsystem zum Leiten eines Lichtstrahls für die Verwendung in Sortieranlage.

Vor diesem Hintergrund liegt der vorliegenden Erfindung die Aufgabe zu Grunde, eine Vorrichtung und ein Verfahren zur Legierungsanalyse von Schrottfragmenten aus Metall zur Verfügung zu stellen, mit denen eine zuverlässigere Analyse einzelner Schrottfragmente ermöglicht wird.

Diese Aufgabe wird bei einer Vorrichtung zur Legierungsanalyse von Schrottfragmenten aus Metall, mit einer Analyseeinrichtung, die dazu eingerichtet ist, mittels einer lokalen Messung an einer Messposition auf der Oberfläche eines zu analysierenden Schrottfragments eine Zusammensetzungsinformation über die Legierungszusammensetzung des Schrottfragments zu bestimmen, erfindungsgemäß zumindest teilweise dadurch gelöst, dass die Vorrichtung weiterhin eine Erfassungseinrichtung aufweist, die dazu eingerichtet ist, einen verunreinigungsarmen oder verunreinigungsfreien Bereich auf der Oberfläche des zu analysierenden Schrottfragments zu bestimmen, und dass die Analyseeinrichtung dazu eingerichtet ist, die Messposition für die lokale Messung in dem von der Erfassungseinrichtung bestimmten Bereich zu positionieren.

Die oben genannte Aufgabe wird erfindungsgemäß weiterhin zumindest teilweise gelöst durch ein Verfahren zur Legierungsanalyse von Schrottfragmenten aus Metall, insbesondere unter Verwendung der zuvor beschriebenen Vorrichtung, bei dem ein verunreinigungsarmer oder verunreinigungsfreier Bereich auf der Oberfläche eines zu analysierenden Schrottfragments bestimmt wird, bei dem eine Messposition in dem bestimmten Bereich ausgewählt wird und bei dem mittels einer lokalen Messung an der ausgewählten Messposition auf der Oberfläche des Schrottfragments eine Zusammensetzungsinformation über die Legierungszusammensetzung des Schrottfragments bestimmt wird.

Durch das beschriebene Verfahren bzw. mit der beschriebenen Vorrichtung wird sichergestellt, dass die Messung zur Bestimmung der Legierungszusammensetzung auf einem möglichst sauberen Bereich der Oberfläche des Schrottfragments erfolgt, so dass das Ergebnis der Legierungsanalyse möglichst gut die Legierungszusammensetzung des Schrottfragments widerspiegelt und nicht zu stark durch Oberflächenverschmutzungen verfälscht wird.

Auf diese Weise kann eine zuverlässige Legierungsanalyse von Schrottfragmenten erreicht werden, mit der insbesondere eine zusammensetzungsspezifische Sortierung der Schrottfragmente ermöglicht wird.

Das Verfahren ist besonders geeignet zur Legierungsanalyse von Schrottfragmenten aus Aluminium bzw. Aluminiumlegierungen.

Die Vorrichtung weist eine Analyseeinrichtung auf, die dazu eingerichtet ist, mittels einer lokalen Messung an einer Messposition auf der Oberfläche eines zu analysierenden Schrottfragments eine Zusammensetzungsinformation über die Legierungszusammensetzung des Schrottfragments zu bestimmen. Die Analyseeinrichtung analysiert also nicht das gesamte Material des Schrottfragments, sondern nur ein Teil des Materials an einer Messposition auf der Oberfläche des Schrottfragments. Eine solche lokale Messung hat den Vorteil, dass sie schnell und relativ einfach durchgeführt werden kann. Weiterhin können für lokale Messungen effektive Messverfahren wie zum Beispiel die Laserinduzierte Plasmaspektroskopie (LIBS) eingesetzt werden, mit denen sich die Zusammensetzung eines Schrottfragments mit hoher Geschwindigkeit und ausreichender Genauigkeit bestimmen lässt.

Die Zusammensetzungsinformation über die Legierungszusammensetzung des Schrottfragments umfasst vorzugsweise einen oder mehrere Werte für den Gehalt eines oder mehrerer Legierungselemente, wie zum Beispiel Magnesium, Silizium, Eisen, Aluminium etc.

Die Vorrichtung weist weiterhin eine Erfassungseinrichtung auf, die dazu eingerichtet ist, einen verunreinigungsarmen oder verunreinigungsfreien Bereich auf der Oberfläche des zu analysierenden Schrottfragments zu bestimmen.

Vorzugsweise ist die Erfassungseinrichtung dazu eingerichtet, einen verunreinigungsfreien Bereich auf der Oberfläche des zu analysierenden Schrottfragments zu bestimmen. In diesem Fall ist die Erfassungseinheit also in der Lage, zwischen verunreinigten und verunreinigungsfreien Bereichen auf der Oberfläche eines Schrottfragments zu unterscheiden. Unter einem verunreinigungsfreien Bereich wird ein Bereich verstanden, in dem das Metall des Schrottfragments freiliegt. Unter einem verunreinigten Bereich wird demgegenüber ein Bereich verstanden, in dem das Metall des Schrottfragments unter einer Beschichtung oder anderweitigen Verunreinigung verborgen ist. Bei der Beschichtung kann es sich insbesondere um eine Lackschicht, eine Eloxierschicht oder eine Kunststoffschicht handeln. Bei den anderweitigen Verunreinigungen kann es sich beispielsweise um Querverschmutzungen aus dem Sammel- und/oder Zerkleinerungsprozess handeln, wie zum Beispiel Stäube oder Schmierstoffe, die an der Oberfläche des Schrottfragments anhaften.

Die Erfassungseinrichtung kann auch dazu eingerichtet sein, einen verunreinigungsarmen Bereich auf der Oberfläche des zu analysierenden Schrottfragments zu bestimmen. Unter einem verunreinigungsarmen Bereich wird ein Bereich verstanden, der gegenüber anderen Bereichen der Oberfläche des Schrottfragments eine geringere Verunreinigung aufweist. In diesem Fall ist die Erfassungseinrichtung also in der Lage zwischen stärker und schwächer verunreinigten Bereichen auf der Oberfläche eines Schrottfragments zu unterscheiden.

Die Analyseeinrichtung ist weiterhin dazu eingerichtet, die Messposition für die lokale Messung in dem von der Erfassungseinrichtung bestimmten Bereich zu positionieren. Auf diese Weise wird erreicht, dass die Messung in einem Bereich durchgeführt wird, in dem das Metall möglichst freiliegt, so dass die Legierungsanalyse weitgehend unverfälschte Zusammensetzungsinformationen liefern kann und sich dadurch die Legierungszusammensetzung des gesamten Schrottfragments möglichst gut bestimmen lässt.

Im Folgenden werden verschiedene Ausführungsformen der Vorrichtung und des Verfahrens beschrieben, wobei die einzelnen Ausführungsformen jeweils sowohl für die Vorrichtung als auch für das Verfahren anwendbar sind und sich zudem untereinander kombinieren lassen

Bei einer ersten Ausführungsform weist die Erfassungseinrichtung eine Bilderfassungseinrichtung zur Aufnahme von Bilddaten der Oberfläche des zu analysierenden Schrottfragments auf sowie eine Auswerteeinrichtung, die dazu eingerichtet ist, einen verunreinigungsarmen oder verunreiningungsfreien Bereich auf der Oberfläche des zu analysierenden Schrottfragments aus den von der Bilderfassungseinrichtung bereitgestellten Bilddaten zu bestimmen. Bei einer entsprechenden Ausführungsform des Verfahrens werden Bilddaten von einem zu analysierenden Schrottfragment aufgenommen und aus den Bilddaten wird der verunreinigungsarme oder verunreinigungsfreie Bereich auf der Oberfläche des zu analysierenden Schrottfragments bestimmt.

Verschmutzungen auf der Oberfläche des Schrottfragments lassen sich gut durch die Analyse von Bilddaten des Schrottfragments erkennen. Beispielsweise können Verschmutzungen wie Lack- oder Kunststoffschichten durch ihre Farbe oder ihre Reflexionseigenschaften relativ einfach von einer sauberen Metalloberfläche unterschieden werden.

Bei der Bilderfassungseinrichtung kann es sich beispielsweise um eine CCD-Kamera, insbesondere um eine Einzeilenkamera, handeln. Als Auswerteeinrichtung kann ein Computer verwendet werden, mit dem die Bilddaten mit bildverarbeitungstechnischen Methoden analysiert werden, um verschmutzte Bereiche von sauberen Bereichen bzw. stärker verschmutzte von weniger stark verschmutzten Bereichen auf der Oberfläche eines Schrottfragments zu unterscheiden.

Die Erfassung von Bilddaten mit der Bilderfassungseinrichtung kann insbesondere im sichtbaren Lichtspektrum erfolgen. Alternativ oder zusätzlich können auch Bilddaten im Infrarot- bzw. Nahinfrarotbereich aufgenommen und analysiert werden. Für die Aufnahme von Bilddaten im Infrarot- bzw. Nahinfrarotbereich ist vorzugsweise eine Infrarot- bzw. Nahinfrarot-Lichtquelle vorgesehen, um das zu analysierende Schrottfragment mit Infrarot- bzw. Nahinfrarotlicht zu beleuchten.

Um aus den aufgenommenen Bilddaten einen verunreinigungsarmen bzw. verunreinigungsfreien Bereich auf der Oberfläche des Schrottfragments zu bestimmen, können insbesondere die Bildpunkte (Pixel) der Bilddaten hinsichtlich ihres Farbwertes in einem Farbraum, beispielsweise dem RGB-Farbraum oder bevorzugt dem YCbCr-Farbraum, bewertet werden. Durch den Vergleich benachbarter Bildpunkte können beispielsweise Änderungen in der Farbe (bei Bilddaten des sichtbaren Lichts) bzw. der Nahinfrarotreflexion (bei Nahinfrarot-Bilddaten) bestimmt werden, aus denen sich einerseits die Außengrenzen des analysierten Schrottfragments und andererseits Grenzen zwischen verunreinigten und verunreinigungsarmen bzw. -freien Bereichen bestimmen lassen, z.B. in Form eines Farbgradienten bzw. Nahinfrarotreflexionsgradienten zwischen benachbarten Pixeln. Auch eine Bewertung der Bildpunkte hinsichtlich ihres Helligkeitswertes ist möglich. Ändert sich zum Beispiel in einem bestimmten Bereich (z.B. an mehreren benachbarten Pixeln) die Farbe, das NIR-Reflexionsspektrum oder die Helligkeit, so ist dies ein Indikator dafür, dass ein Übergang zu einem anderen Bereich vorliegt (insbesondere von einem verunreinigten zu einem verunreinigungsarmen/-freien Bereich).

Zur Feststellung, ob es sich bei einem identifizierten Bereich um einen verunreinigungsarmen bzw. -freien Bereichen handelt, erfolgt vorzugsweise ein Vergleich der Bilddaten oder aus den Bilddaten abgeleiteter Größen mit Referenzwerten. Für einen Vergleich der Bilddaten mit Referenzwerten können beispielsweise in einem Bereich gemessene Helligkeits- oder Farbwerte mit einem oder mehreren Referenzwerten verglichen werden.

Zur Feststellung, ob es sich bei einem identifizierten Bereich um einen verunreinigungsarmen bzw. -freien Bereichen handelt, erfolgt vorzugsweise ein Vergleich des in diesem Bereich gemessenen Farbwerts oder Nahinfrarotspektrums mit einem Referenzwert.

Entsprechend erfolgt bei einer Ausführungsform des Verfahrens bzw. der Vorrichtung die Bestimmung eines verunreinigungsarmen oder verunreiningungsfreien Bereichs auf der Oberfläche des zu analysierenden Schrottfragments aus den von der Bilderfassungseinrichtung bereitgestellten Bilddaten durch Vergleich von Farb- und/oder Helligkeitswerten aus einem Bereich der Bilddaten mit Referenzwerten. Für den Vergleich können beispielsweise die Farb- und/oder Helligkeitswertverteilung in einem Bereich der Bilddaten bestimmt werden, beispielsweise in Form eines Histogramms, und mit Referenzwerten verglichen werden. So können zum Beispiel der Mittelwert oder die Varianz der für den Bereich bestimmten Farb- und/oder Helligkeitswertverteilung mit entsprechenden Referenzwerten für den Mittelwert bzw. die Varianz verglichen werden.

Zur Bestimmung eines verunreinigungsarmen oder verunreiningungsfreien Bereichs können weiterhin auch Formen in den Bilddaten erkannt und mit Referenzformen verglichen werden. Bei den Formen in den Bilddaten handelt es sich insbesondere um Bildpunkt-Bereiche mit ähnlichen Eigenschaften (z.B. ähnlicher Farbe und/oder Helligkeit etc.). Beispielsweise ist es denkbar, anhand von räumlichen Änderungen in den Farb- und/oder Helligkeitswerten der Bilddaten auf das Vorhandensein oder Nichtvorhandensein bzw. auf die Größe von Formparametern von Formen geschlossen werden, die mit Referenzwerten über das Vorhandensein bestimmter Formen oder mit Referenzwerten für Formparameter verglichen werden. Anhand von Änderungen in den Farb- und/oder Helligkeitswerten lassen sich insbesondere die Ränder von Formen bestimmen. Als Formen kommen beispielsweise Kreise, Dreiecke, Vierecke etc. in Betracht. Als Formparameter kommen beispielsweise Durchmesser von Kreisen, Winkel, Seitenlängen und/oder Seitenverhältnisse von Drei- oder Vierecken in Betracht. Die Information über das Vorhandensein einer bestimmten Form oder Werte der zuvor beschriebenen Formparameter stellen Beispiele für aus den Bilddaten abgeleitete Größen dar.

Zur Ermittlung entsprechender Referenzwerte können insbesondere eine Referenzprobe in der Vorrichtung angeordnet und mit der Bilderfassungseinrichtung Bilddaten von der Referenzprobe erfasst werden. Als Referenzprobe kann beispielsweise ein Metallstück mit einer zumindest bereichsweisen verunreinigungsfreien Oberfläche verwendet werden. Das Metallstück weist vorzugsweise eine den zu analysierenden Schrottfragmenten vergleichbare Zusammensetzung auf. Sollen mit der Vorrichtung beispielsweise Aluminiumschrottfragmente analysiert werden, so wird als Referenzprobe insbesondere ein Aluminiumstück mit sauberer Oberfläche verwendet.

Da von der Referenzprobe und dem zu analysierenden Schrottfragment im Wesentlichen unter gleichen Bedingungen Bilddaten aufgenommen werden, kann durch einen Vergleich der Bilddaten der Referenzprobe bzw. eines daraus bestimmten Referenzwertes mit den Bilddaten des zu analysierenden Schrottfragments ein verunreinigungsarmer bzw. -freier Bereich auf der Oberfläche des Schrottfragments bestimmt werden. Die Aufnahme von Bilddaten an der Referenzprobe dient damit insbesondere zur Kalibration der Erfassungseinrichtung.

Durch einen solchen Vergleich mit einem Referenzwert bzw. den Bilddaten einer Referenzprobe ist es z.B. nicht erforderlich, einen Farbwert von der Schrottfragmentoberfläche absolut zu bestimmen. Insbesondere muss die Messung nicht unter genormten Bedingungen (z.B. unter Normlicht mit vorgegebenen Normalbeobachter etc.) erfolgen, da es nur auf einen relativen Vergleich mit Bilddaten der Referenzprobe ankommt, nicht aber auf absolute Farbwerte. Die Bilddaten an der Referenzprobe soll jedoch unter im Wesentlichen gleichen Bedingungen aufgenommen werden, wie die Bilddaten der zu analysierenden Schrottfragmente, um eine möglichst zuverlässige Bestimmung des verunreiningungsarmen bzw. -freien Bereichs zu ermöglichen.

Die Referenzwerte werden vorzugsweise mittels mehrerer Referenzproben in Form mehrerer Schrottfragmente bestimmt. Beispielsweise können Referenzwerte durch Mittelwertbildung von Werten verschiedener Referenzproben bestimmt werden.

Zusätzlich oder alternativ zum Vergleich mit einem Referenzwert kann ein Farbwert, Nahinfrarotspektrum oder Helligkeitswert aus einem in den Bilddaten bestimmten Bereich auch mit einem entsprechenden Farbwert, Nahinfrarotspektrum bzw. Helligkeitswert aus einem anderen in den Bilddaten bestimmten Bereich verglichen werden. Durch einen solchen relativen Vergleich zwischen verschiedenen Bereichen innerhalb der Bilddaten können ebenfalls verunreinigungsarme bzw. -freie von verunreinigten Bereichen unterschieden werden.

Entsprechend erfolgt bei einer Ausführungsform des Verfahrens bzw. der Vorrichtung die Bestimmung eines verunreinigungsarmen oder verunreiningungsfreien Bereichs auf der Oberfläche des zu analysierenden Schrottfragments aus den von der Bilderfassungseinrichtung bereitgestellten Bilddaten durch Vergleich von Farb- und/oder Helligkeitswerten aus einem Bereich der Bilddaten mit Farb- und/oder Helligkeitswerten aus einem anderen Bereich der Bilddaten. Für einen solchen Vergleich können insbesondere die Farb- und/oder Helligkeitswertverteilungen in den jeweiligen Bereichen bestimmt und miteinander verglichen werden, beispielsweise durch Vergleich der jeweiligen Mittelwerte oder Varianzen der Farb- bzw. Helligkeitswertverteilungen.

Bei einer Ausführungsform umfasst das Bestimmen eines verunreinigungsarmen oder verunreinigungsfreien Bereichs auf der Oberfläche eines zu analysierenden Schrottfragments folgende Schritte bzw. die Analyseeinrichtung ist zur Durchführung der folgenden Schritte eingerichtet:
- Bestimmen von verschiedenen Bereichen in den Bilddaten, wobei ein Bereich jeweils eine Menge zusammenhängender Bildpunkte mit ähnlichen Farb-, Nahinfrarot- und/oder Helligkeitswerten umfasst,
- Identifizieren eines verunreinigungsfreien bzw. verunreinigungsarmen Bereichs abhängig von den Farb-, Nahinfrarot- und/oder Helligkeitswerten der Bildpunkte des Bereichs, insbesondere durch Vergleich mit einem Referenzwert oder den entsprechenden Werten eines anderen Bereichs.

Die Aufnahme und Auswertung von Bilddaten im sichtbaren Lichtspektrum ist besonders gut geeignet, um farbige und insbesondere dunkel lackierte (d.h. verunreinigte) Bereiche auf einem Schrottfragment von verunreinigungsfreien Bereichen zu unterscheiden. Schwierig ist die Unterscheidung zwischen lackierten und verunreinigungsfreien Bereichen hingegen bei Klar- oder Silberlacken. Diese lassen sich hingegen gut in Bilddaten im Nahinfrarot von verunreinigungsfreien Bereichen unterscheiden. Demgegenüber sind Bereiche mit dunklen Lacken wiederum schwierig in Nahinfrarot-Bilddaten identifizierbar.

Bilddaten im sichtbaren Lichtspektrum und im Nahinfrarot-Spektrum liefern demnach komplementäre Informationen, die sich bei der Bestimmung eines verunreinigungsarmen bzw. -freien Bereichs vorteilhaft ergänzen. Entsprechend ist in einer vorteilhaften Ausführungsform die Bilderfassungseinrichtung dazu eingerichtet Bilddaten im sichtbaren Bereich und im Nahinfrarotbereich aufzunehmen und die Auswerteeinrichtung ist dazu eingerichtet, aus den Bilddaten im sichtbaren Bereich und im Nahinfrarotbereich einen verunreinigungsarmen oder -freien Bereich auf der Oberfläche des zu analysierenden Schrottfragments zu bestimmen. Zu diesem Zweck kann die Bilderfassungseinrichtung beispielsweise zwei Kameras umfassen, von denen eine für den sichtbaren Bereich und eine auf den Nahinfrarotbereich eingerichtet ist.

Bei einer entsprechenden Ausführungsform des Verfahrens werden Bilddaten im sichtbaren Lichtspektrum und Bilddaten im Nahinfrarot-Spektrum von einem zu analysierenden Schrottfragment aufgenommen und der verunreinigungsarme oder - freie Bereich auf der Oberfläche des zu analysierenden Schrottfragments wird aus den Bilddaten im sichtbaren Lichtspektrum und den Bilddaten im Nahinfrarot-Spektrum bestimmt.

Die Kamera zur Erfassung der Bilddaten im sichtbaren Lichtspektrum und die Kamera zur Erfassung von Bilddaten im Nahinfrarotbereich können beispielsweise so ausgerichtet werden, dass sich die von den Kameras erfassten Aufnahmebereiche zumindest teilweise überlappen. Auf diese Weise kann ein Schrottfragment gleichzeitig im Aufnahmebereich beider Kameras angeordnet sein. Vorzugsweise sind die Kameras in diesem Fall so synchronisiert, dass die Bilddaten im sichtbaren Lichtspektrum und die Bilddaten im Nahinfrarot-Spektrum im Wesentlichen gleichzeitig erfasst werden. Mit der bekannten Positionierung und Ausrichtung der beiden Kameras können die Bildpunkte der Bilddaten von einer der Kameras mittels einer vorgegebenen Zuordnungsfunktion einfach den Bildpunkten der Bilddaten von der anderen Kamera zugeordnet werden, die identischen räumlichen Positionen auf dem erfassten Schrottfragment entsprechen.

Die Kamera für das sichtbare Lichtspektrum und die Kamera für den Nahinfrarotbereich können auch so ausgerichtet werden, dass sie im Wesentlichen disjunkte, insbesondere in Transportrichtung der zu analysierenden Schrottfragmente voneinander beabstandete Aufnahmebereiche haben. In diesem Fall sind die Kameras vorzugsweise so synchronisiert, dass die Zeit zwischen der Erfassung der Bilddaten im sichtbaren Spektrum und der Erfassung der Bilddaten im Nahinfrarot-Spektrum im Wesentlichen der Zeitdauer entspricht, in der ein zu analysierendes Schrottfragment aus dem Aufnahmebereich der in Transportrichtung zuerst angeordneten Kamera in den Aufnahmebereich der anderen Kamera transportiert wird. Durch die bekannte Positionierung und Ausrichtung der beiden Kameras und die bekannte Transportgeschwindigkeit der Schrottfragmente können die Bildpunkte der Bilddaten von einer der Kameras mittels einer vorgegebenen Zuordnungsfunktion einfach den Bildpunkten der Bilddaten der anderen Kamera zugeordnet werden, die identischen räumlichen Positionen auf dem erfassten Schrottfragment entsprechen.

Die räumliche Trennung der Aufnahmebereiche der Kamera für das sichtbaren Lichtspektrum und der Kamera für den Nahinfrarotbereich erlaubt eine unterschiedliche und vorzugsweise an die jeweilige Kamera angepasste Ausleuchtung des Aufnahmebereichs. Beispielsweise kann für einen der Aufnahmebereiche oder für beide Aufnahmebereiche jeweils eine Lichtquelle vorgesehen sein, die den Aufnahmebereich angepasst an die jeweilige Kamera ausleuchtet. So kann zum Beispiel für den Aufnahmebereich der Kamera für den Nahinfrarotbereich eine Lichtquelle mit hohem Infrarotanteil und/oder für den Aufnahmebereich der Kamera für das sichtbare Lichtspektrum eine Lichtquelle mit hohem Anteil im sichtbaren Lichtspektrum verwendet werden. Dadurch kann die Qualität der Bilddaten verbessert werden. Weiterhin kann ein Übersteuern beispielsweise der Bilddaten im Nahinfrarotbereich durch starke Lichtanteile im sichtbaren Bereich verhindert werden.

Bei einer Ausführungsform werden in einem Sichtbar-Auswertungsschritt ein oder mehrere Sichtbar-Kandidatenbereiche für verunreinigungsarme oder -freie Bereiche in den Bilddaten im sichtbaren Lichtspektrum bestimmt und in einem Nahinfrarot-Auswertungsschritt ein oder mehrere Nahinfrarot-Kandidatenbereiche für verunreinigungsarme oder -freie Bereiche in den Bilddaten im Nahinfrarot-Spektrum bestimmt. Der verunreinigungsarme oder -freie Bereich auf der Oberfläche des zu analysierenden Schrottfragments kann dann abhängig von den Sichtbar-Kandidatenbereichen und den Nahinfrarot-Kandidatenbereichen bestimmt werden. Insbesondere kann der verunreinigungsarme oder -freie Bereich so ausgewählt werden, dass er in einem Sichtbar-Kandidatenbereich und in einem Nahinfrarot-Kandidatenbereich liegt.

Beispielsweise können anhand der Bilddaten im sichtbaren Spektrum ein oder mehrere Sichtbar-Kandidatenbereiche für verunreinigungsarme oder -freie Bereiche bestimmt werden. Anhand der Bilddaten im Nahinfrarot-Spektrum kann dann bestimmt werden, ob in dem einen oder den mehreren Sichtbar-Kandidatenbereichen eine organische Beschichtung, zum Beispiel ein durchsichtiger in den Bilddaten im sichtbaren Spektrum nicht sichtbarer Lack, vorhanden ist. Als verunreinigungsarmer bzw. -freier Bereich kann dann zum Beispiel einer der Sichtbar-Kandidatenbereiche ausgewählt werden, in dem kein organischer Lack vorhanden ist.

Die aus den Bilddaten ermittelbare Form von Bereichen mit ähnlicher Farbe, ähnlicher Helligkeit bzw. ähnlichem Nahinfrarotspektrum kann bei der Auswertung ebenfalls verwendet werden, um einen verunreinigungsarmen bzw. -freien Bereich zu identifizieren. Ändert sich in einem bestimmten Bereich der Bilddaten, beispielsweise an mehreren benachbarten Bildpunkten, die Helligkeit bzw. das Nahinfrarotspektrum, so ist dies ein Indikator dafür, dass der betreffende Bereich verunreinigungsfrei oder verunreinigungsarm ist.

Weiterhin kann aus den Formen von Bildpunkt-Bereichen mit ähnlichen Eigenschaften (z.B. Farbe, Helligkeit etc.) auf verunreinigungsfreie bzw. -arme Bereiche geschlossen werden. Insbesondere lässt sich aus den Formen zunächst bestimmen, welche Bereiche in den Bilddaten zum Förderband bzw. zu dem auf dem Förderband transportierten Schrottfragment gehören. Mit dieser Information lässt sich dann weiter bestimmen, welche Bereiche innerhalb des Schrottfragments liegen und daher als verunreinigungsfreie bzw. -arme Bereiche des Schrottfragments in Betracht kommen. Auf diese Weise können Fehlzuordnungen vermieden werden.

Nach der Identifizierung eines verunreinigungsarmen bzw. -freien Bereichs wählt die Analyseeinrichtung vorzugsweise eine Position innerhalb dieses Bereichs als Messposition aus. Über die Bildpunktkoordinaten in den Bilddaten kann die Position in den Bilddaten einer tatsächlichen Position auf der Oberfläche der Schrottfragmentoberfläche zugewiesen werden. Zu diesem Zweck weist die Bilderfassungseinrichtung insbesondere eine definierte Position zur Position des von der Bilderfassungseinrichtung aufgenommenen Bereichs auf, so dass eine Umrechnung von Bilddatenkoordinaten in reale Koordinaten, beispielsweise auf der Oberfläche eines Förderbands, möglich ist.

Bei einer weiteren Ausführungsform ist die Analyseeinrichtung für eine spektroskopische Messung eingerichtet, insbesondere für eine laserinduzierte Plasmaspektroskopiemessung (LIBS). Bei einer entsprechenden Ausführungsform des Verfahrens ist die lokale Messung an der ausgewählten Messposition auf der Oberfläche des Schrottfragments eine spektroskopische Messung, insbesondere eine laserinduzierte Plasmaspektroskopiemessung. Durch eine spektroskopische Messung lässt sich auf schnelle Weise die Legierungszusammensetzung an der Oberfläche eines Schrottfragments bestimmen. Die laserinduzierte Plasmaspektroskopie hat sich als besonders geeignetes Verfahren herausgestellt, um die Legierungszusammensetzung schnell und zuverlässig zu messen. Da das LIBS-Verfahren oberflächensensitiv ist, d.h. die Zusammensetzung nur im Oberflächenbereich des Schrottfragments bestimmen kann, wird eine LIBS-Messung stark von etwaigen Verschmutzungen der Oberfläche beeinflusst. Durch das vorliegend beschriebene Verfahren bzw. die vorliegend beschriebene Vorrichtung wird erreicht, dass mit einer LIBS-Messung möglichst unverfälschte Ergebnisse über die Legierungszusammensetzung des Schrottfragments bestimmt werden können.

Bei einer weiteren Ausführungsform weist die Analyseeinrichtung eine Positionierungseinrichtung auf, um einen Laserstrahl auf eine ausgewählte Messposition zu richten. Bei einer entsprechenden Ausführungsform des Verfahrens wird zur Durchführung der Messung ein Laserstrahl auf die ausgewählte Messposition gerichtet. Eine lokale Messung der Legierungszusammensetzung des Schrottfragments kann insbesondere durch Laserablation erfolgen, wobei mit einem Laserstrahl etwas Material von der Oberfläche des Schrottfragments verdampft und dann vorzugsweise optisch analysiert wird. Dieses Verfahren wird beispielsweise bei der laserinduzierten Plasmaspektroskopie eingesetzt. Die Messposition entspricht bei einem solchen Verfahren der Stelle, an der der Laserstrahls auf der Oberfläche auftrifft. Daher lässt sich mit der zuvor beschriebenen Positionierungseinrichtung die Messposition auf der Oberfläche des Schrottfragments genau einstellen, um eine lokale Messung in einem verunreiningungsarmen oder verunreinigungsfreien Bereich sicherzustellen. Die Positionierungseinrichtung kann beispielsweise eine bewegliche Optik oder Spiegel umfassen, mit denen sich der Laserstrahl auf eine bestimmte Stelle auf der Oberfläche des Schrottfragments richten lässt. Alternativ kann die Positionierungseinrichtung auch dazu eingerichtet sein, die Position eines Schrottfragments relativ zum Laserstrahl auszurichten, so dass der Laserstrahl auf die gewünschte Messposition auftrifft.

Bei einer weiteren Ausführungsform umfasst die Vorrichtung eine Fördereinrichtung zum Transport von Schrottfragmenten durch einen Analysebereich, in dem die Messung durch die Analyseeinrichtung erfolgt. Bei einer entsprechenden Ausführungsform des Verfahrens wird ein zu analysierendes Schrottfragment durch einen Analysebereich transportiert, in dem die Messung an dem Schrottfragment erfolgt. Das Verfahren und die Vorrichtung können insbesondere zur Analyse einer Menge Schrottfragmente eingesetzt werden, beispielsweise um eine Menge Schrottfragmente legierungsspezifisch zu sortieren. Mit der Fördereinrichtung können die Schrottfragmente nach und nach zur Analyseeinrichtung transportiert und dort sukzessive analysiert werden. Bei der Fördereinrichtung kann es sich beispielsweise um ein Förderband handeln.

Bei einer weiteren Ausführungsform umfasst die Vorrichtung eine Vereinzelungseinrichtung, die dazu eingerichtet ist, eine Menge Schrottfragmente zu vereinzeln, so dass die Schrottfragmente der Analyseeinrichtung vereinzelt zugeführt werden. Bei einer entsprechenden Ausführungsform des Verfahrens wird eine bereitgestellte Menge Schrottfragmente vereinzelt und für die vereinzelten Schrottfragmente werden Zusammensetzungsinformationen bestimmt. Durch die Vereinzelung der Schrottfragmente wird erreicht, dass die einzelnen Schrottfragmente zuverlässig analysiert werden können, ohne dass Schrottfragmente einander überlappen. Weiterhin wird durch die Vereinzelung die Bestimmung eines verunreinigungsarmen oder verunreinigungsfreien Bereichs auf der Oberfläche der jeweiligen Schrottfragmente erleichtert. Durch eine Vereinzelung können die Schrottfragmente zudem in einer wohldefinierten Reihenfolge analysiert werden, wodurch die Zuordnung der bestimmten Zusammensetzungsinformationen zu den einzelnen Schrottfragmenten, beispielsweise für eine nachgelagerte Sortierung der Schrottfragmente, vereinfacht wird.

Bei einer weiteren Ausführungsform umfasst die Vorrichtung eine Sortiereinrichtung, die dazu eingerichtet ist, Schrottfragmente abhängig von der jeweils bestimmten Zusammensetzungsinformation zu sortieren. Bei einer entsprechenden Ausführungsform des Verfahrens werden Schrottfragmente abhängig von der jeweils bestimmten Zusammensetzungsinformation sortiert. Die für die einzelnen Schrottfragmente bestimmten Zusammensetzungsinformationen erlauben es, die Schrottfragmente legierungsspezifisch zu sortieren. Insbesondere kann durch die Messung in einem verunreinigungsarmen oder verunreinigungsfreien Bereich auf der Oberfläche der einzelnen Schrottfragmente eine bessere Sortierung erreicht werden, so dass sich die beschriebene Legierungsanalyse insbesondere für ein Verfahren bzw. eine Vorrichtung zur Sortierung von Schrottfragmenten eignet.

Bei einer weiteren Ausführungsform weist die Vorrichtung eine Steuerungseinrichtung auf, die dazu eingerichtet ist, die Vorrichtung gemäß dem zuvor beschriebenen Verfahren bzw. einer Ausführungsform davon zu steuern. Die Steuerungseinrichtung kann beispielsweise einen Mikroprozessor und einen damit verbunden elektronischen Speicher aufweisen, wobei der Speicher Befehle enthält, deren Ausführung auf dem Mikroprozessor die Ausführung des beschriebenen Verfahrens bzw. einer Ausführungsform davon bewirkt. Auf diese Weise lässt sich die Analyse der Schrottfragmente automatisiert und zuverlässig durchführen.

Im Folgenden werden weitere Ausführungsformen 1 bis 8 der Vorrichtung und 9 bis 14 des Verfahrens beschrieben. Diese können insbesondere auch mit den zuvor beschriebenen Merkmalen kombiniert werden.
1. Vorrichtung zur Legierungsanalyse von Schrottfragmenten aus Metall, mit einer Analyseeinrichtung, die dazu eingerichtet ist, mittels einer lokalen Messung an einer Messposition auf der Oberfläche eines zu analysierenden Schrottfragments eine Zusammensetzungsinformation über die Legierungszusammensetzung des Schrottfragments zu bestimmen, dadurch gekennzeichnet, dass die Vorrichtung eine Erfassungseinrichtung aufweist, die dazu eingerichtet ist, einen verunreinigungsarmen oder verunreinigungsfreien Bereich auf der Oberfläche des zu analysierenden Schrottfragments zu bestimmen, und dass die Analyseeinrichtung dazu eingerichtet ist, die Messposition für die lokale Messung in dem von der Erfassungseinrichtung bestimmten Bereich zu positionieren.
2. Vorrichtung nach Ausführungsform 1, dadurch gekennzeichnet, dass die Erfassungseinrichtung eine Bilderfassungseinrichtung zur Aufnahme von Bilddaten der Oberfläche des zu analysierenden Schrottfragments aufweist sowie eine Auswerteeinrichtung, die dazu eingerichtet ist, einen verunreinigungsarmen oder verunreinigungsfreien Bereich auf der Oberfläche des zu analysierenden Schrottfragments aus den von der Bilderfassungseinrichtung bereitgestellten Bilddaten zu bestimmen, insbesondere durch Vergleich von Farb- und/oder Helligkeitswerten aus einem Bereich der Bilddaten mit Farb- und/oder Helligkeitswerten aus einem anderen Bereich der Bilddaten oder mit Referenzwerten.
3. Vorrichtung nach Ausführungsform 1 oder 2, dadurch gekennzeichnet, dass die Analyseeinrichtung für eine spektroskopische Messung eingerichtet ist, insbesondere für eine laserinduzierte Plasmaspektroskopiemessung.
4. Vorrichtung nach einer der Ausführungsformen 1 bis 3, dadurch gekennzeichnet, dass die Analyseeinrichtung eine Positionierungseinrichtung aufweist, um einen Laserstrahl auf eine ausgewählte Messposition zu richten.
5. Vorrichtung nacheiner der Ausführungsformen 1 bis 4, weiter umfassend eine Fördereinrichtung zum Transport von Schrottfragmenten durch einen Analysebereich, in dem die Messung durch die Analyseeinrichtung erfolgt.
6. Vorrichtung nach einer der Ausführungsformen 1 bis 5, weiter umfassend eine Vereinzelungseinrichtung, die dazu eingerichtet ist, eine Menge Schrottfragmente zu vereinzeln, so dass die Schrottfragmente der Analyseeinrichtung vereinzelt zugeführt werden.
7. Vorrichtung nach einer der Ausführungsformen 1 bis 6, weiter umfassend eine Sortiereinrichtung, die dazu eingerichtet ist, Schrottfragmente abhängig von der jeweils bestimmten Zusammensetzungsinformation zu sortieren.
8. Vorrichtung nach einer der Ausführungsformen 1 bis 7, aufweisend eine Steuerungseinrichtung, die dazu eingerichtet ist, die Vorrichtung gemäß einem Verfahren nach einer der Ausführungsformen 9 bis 14 zu steuern.
9. Verfahren zur Legierungsanalyse von Schrottfragmenten aus Metall, insbesondere unter Verwendung einer Vorrichtung nach einer der Ausführungsformen 1 bis 8, bei dem ein verunreinigungsarmer oder verunreinigungsfreier Bereich auf der Oberfläche eines zu analysierenden Schrottfragments bestimmt wird, bei dem eine Messposition in dem bestimmten Bereich ausgewählt wird und bei dem mittels einer lokalen Messung an der ausgewählten Messposition auf der Oberfläche des Schrottfragments eine Zusammensetzungsinformation über die Legierungszusammensetzung des Schrottfragments bestimmt wird.
10. Verfahren nach Ausführungsform 9, dadurch gekennzeichnet, dass Bilddaten von einem zu analysierenden Schrottfragment aufgenommen werden und der verunreinigungsfreie Bereich auf der Oberfläche des zu analysierenden Schrottfragments aus den Bilddaten bestimmt wird, insbesondere durch Vergleich von Farb- und/oder Helligkeitswerten aus einem Bereich der Bilddaten mit Farb- und/oder Helligkeitswerten aus einem anderen Bereich der Bilddaten oder mit Referenzwerten.
11. Verfahren nach Ausführungsform 9 oder 10, dadurch gekennzeichnet, dass zur Durchführung der Messung ein Laserstrahl auf die ausgewählte Messposition gerichtet wird.
12. Verfahren nach einer der Ausführungsformen 9 bis 11, dadurch gekennzeichnet, dass die lokale Messung an der ausgewählten Messposition auf der Oberfläche des Schrottfragments eine spektroskopische Messung ist, insbesondere eine laserinduzierte Plasmaspektroskopiemessung.
13. Verfahren nach einer der Ausführungsformen 9 bis 12, dadurch gekennzeichnet, dass eine bereitgestellte Menge Schrottfragmente vereinzelt wird und für die vereinzelten Schrottfragmente Zusammensetzungsinformationen bestimmt werden.
14. Verfahren nach einer der Ausführungsformen 9 bis 13, dadurch gekennzeichnet, dass Schrottfragmente abhängig von der jeweils bestimmten Zusammensetzungsinformation sortiert werden.

Weitere Vorteile und Merkmale der Vorrichtung und des Verfahrens ergeben sich aus der nachfolgenden Beschreibung von Ausführungsbeispielen, wobei auf die beigefügte Zeichnung Bezug genommen wird.

In der Zeichnung zeigen
- Fig. 1: ein Ausführungsbeispiel der erfindungsgemäßen Vorrichtung und des erfindungsgemäßen Verfahrens in schematischer Darstellung,
- Fig. 2: die Analyseeinrichtung der Vorrichtung aus Fig. 1,
- Fig. 3: eine Illustration von Bilddaten eines Schrottfragments,
- Fig. 4: ein Beispiel für Bilddaten eines Schrottfragments,
- Fig. 5a-c: Histogramm-Analysen von Bilddaten für einen verunreinigungsarmen Bereich des Schrottfragments aus Fig. 4 und
- Fig. 6a-c: Histogramm-Analysen von Bilddaten für einen verunreinigten Bereich des Schrottfragments aus Fig. 4.

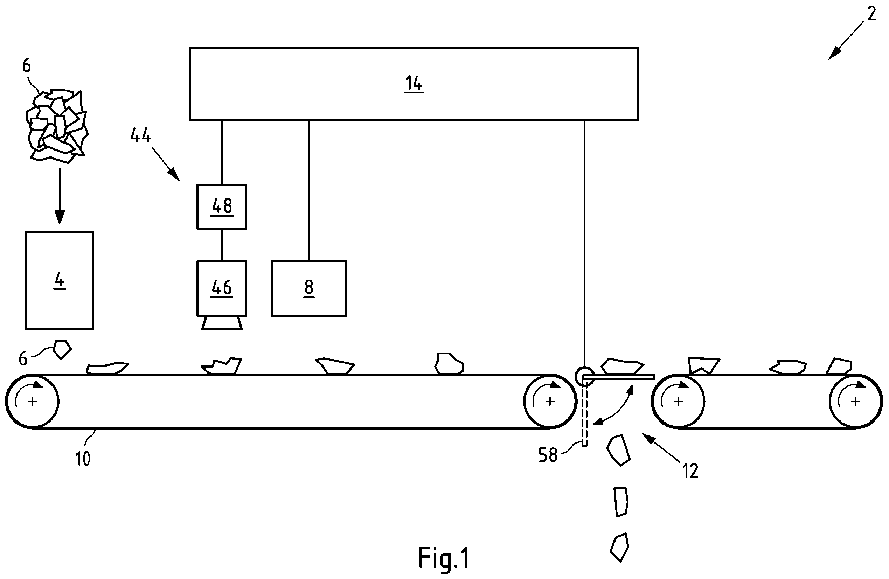

Fig. 1 zeigt ein Ausführungsbeispiel der erfindungsgemäßen Vorrichtung und des erfindungsgemäßen Verfahrens zur Legierungsanalyse von Schrottfragmenten aus Metall in schematischer Darstellung.

Die Vorrichtung 2 umfasst eine Vereinzelungseinrichtung 4 zur Vereinzelung von Schrottfragmenten 6, eine Analyseeinrichtung 8 zur Legierungsanalyse der Schrottfragmente 6 sowie eine Fördereinrichtung 10 in Form eines Förderbands, mit dem die Schrottfragmente 6 durch die Vorrichtung 2 transportiert werden. Die Vorrichtung 2 kann wie in Fig. 1 dargestellt als Sortiervorrichtung ausgebildet sein und entsprechend eine Sortiereinrichtung 12 umfassen. Weiterhin weist die Vorrichtung noch eine Steuerungseinrichtung 14 zur Steuerung der Vorrichtung 2 auf.

Wird der Vereinzelungseinrichtung 4 eine Menge Schrottfragmente 6 zugegeben, so werden die Schrottfragmente 6 durch die Vereinzelungseinrichtung 4 vereinzelt und so auf die Fördereinrichtung 10 gegeben, dass sie einander nicht überlappen. Weiterhin weisen die Schrottfragmente 6 durch die Vereinzelung eine wohldefinierte Reihenfolge auf, wodurch die Analyse und eine mögliche anschließende Sortierung der Schrottfragmente 6 erleichtert wird.

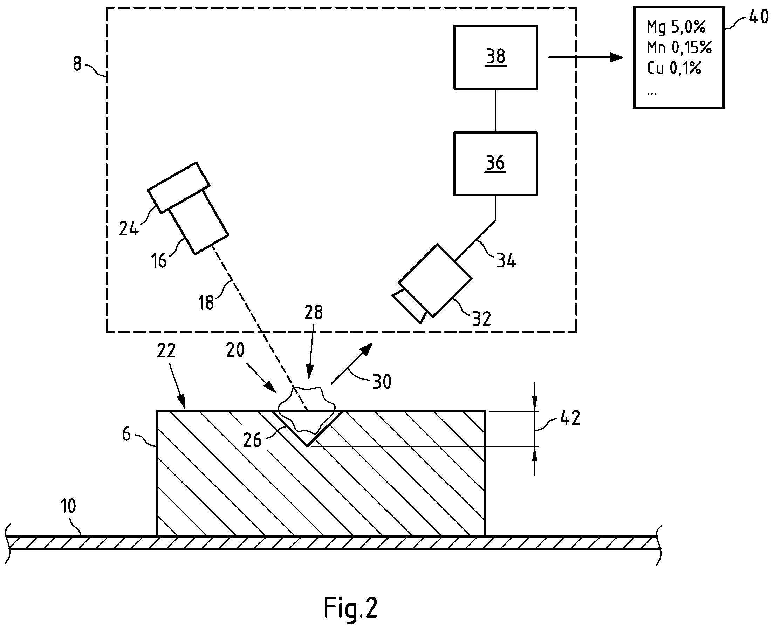

Die auf der Fördereinrichtung 10 transportierten Schrottfragmente 6 werden dann einzeln von der Analyseeinrichtung 8 mittels laserinduzierter Plasmaspektroskopie untersucht, um die Legierungszusammensetzung der einzelnen Schrottfragmente 6 zu bestimmen. Der Aufbau und die Funktionsweise der Analyseeinrichtung 8 werden im Folgenden anhand der Fig. 2 erläutert, die eine detailliertere schematische Darstellung der Analyseeinrichtung 8 zeigt.

Die Analyseeinrichtung 8 umfasst eine Laserquelle 16, mit der ein gepulster Laserstrahl 18 auf eine Messposition 20 auf der Oberfläche 22 eines auf der Fördereinrichtung 10 beförderten Schrottfragments 6 gerichtet werden kann. Um die Messposition 20 auf der Oberfläche 22 gezielt einstellen zu können, umfasst die Analyseeinrichtung 8 eine Positionierungseinrichtung 24, mit der sich der Laserstrahl 18 ausrichten lässt.

Durch den auf der Oberfläche 22 auftreffenden Laserstrahl 18 wird an der Messposition 20 ein kleines Volumen 26 im Bereich der Oberfläche des Schrottfragments 6 verdampft und zu einem Plasma 28 ionisiert. Beim Zerfall des Plasmas 28 wird Licht 30 emittiert, das charakteristisch für die im Volumen 26 enthaltenen Legierungselemente ist. Durch eine spektroskopische Analyse des Lichts 30 lassen sich daher die Legierungselemente im Plasma 28 bestimmen und damit auf die Legierungszusammensetzung des Volumens 26 rückschließen.

Entsprechend weist die Analyseeinrichtung 8 weiterhin eine Optik 32 auf, um das Licht 30 einzufangen und über einen Lichtleiter 34 an ein Spektrometer 36 weiterzuleiten, mit dem die Spektralverteilung des Lichts 30 analysiert werden kann. Eine an das Spektrometer angeschlossene Auswerteelektronik 38 berechnet dann aus der gemessenen Spektralverteilung die Legierungszusammensetzung im Plasma 28 und damit des Volumens 26 und gibt eine entsprechende Zusammensetzungsinformation 40 aus, die insbesondere die Gehalte bestimmter Legierungselemente wie Mg, Mn, Cu etc. im Volumen 26 in Gew.-% enthalten kann.

Da der Laserstrahl 18 auf eine bestimmte Messposition 20 gerichtet ist und zudem nur eine bestimmte Eindringtiefe 42 hat, die abhängig von der eingestellten Laserleistung typischerweise im Bereich von 1 bis 100 µm liegt, führt die Analyseeinrichtung 8 also eine lokale Messung an einer Messposition 20 auf der Oberfläche 22 des Schrottfragments 6 durch.

Befinden sich an der Messposition 20 Verunreinigungen wie zum Beispiel eine Lackschicht, eine Kunststoffschicht oder Schmierstoffe, so wird die Messung hierdurch stark verfälscht, so dass die Zusammensetzungsinformation 40 keine zuverlässigen Informationen über die tatsächliche Legierungszusammensetzung des Schrottfragments 6 liefert.

Aus diesem Grund weist die in Fig. 1 gezeigte Vorrichtung 2 zusätzlich eine Erfassungseinrichtung 44 auf, mit der ein verunreinigungsarmer oder verunreinigungsfreier Bereich auf der Oberfläche 22 des zu analysierenden Schrottfragments 6 bestimmt werden kann.

Die Erfassungseinrichtung 44 weist eine Kamera 46 auf, die auf die Fördereinrichtung 10 gerichtet ist und auf diese Weise Bilddaten von den Oberflächen 22 der auf der Förderreinrichtung 10 transportierten Schrottfragmente 6 erfassen kann. Weiterhin weist die Erfassungseinrichtung 44 eine Auswerteeinrichtung 48 auf, mit der die Bilddaten von den Oberflächen 22 der Schrottfragmente 6 analysiert werden können, um einen verunreinigungsarmen oder verunreinigungsfreien Bereich auf den Oberflächen 22 zu bestimmen.

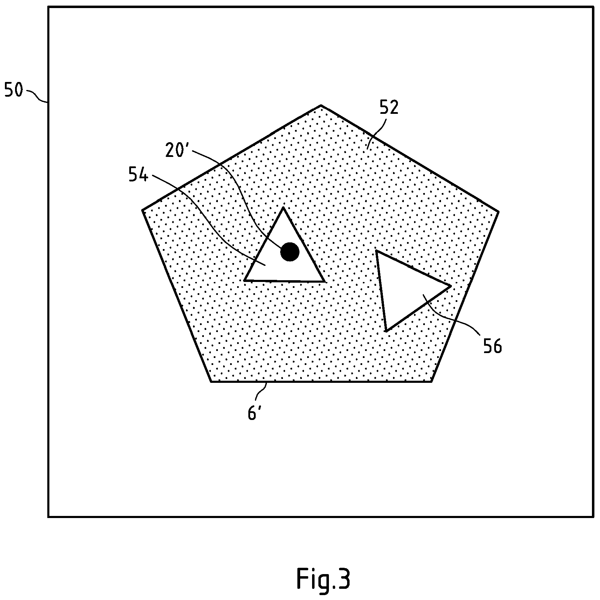

Fig. 3 zeigt ein schematisches Beispiel eines Bilds 50, das mit der Kamera 46 von der Oberfläche 22 eines Schrottfragments 6' aufgenommen wurde. Bei dem Schrottfragment 6' handelt es sich um ein Aluminiumschrottfragment aus einer lackierten Kraftfahrzeugtür. Das Schrottfragment 6' weist an der Oberfläche einen Bereich 52 auf, in dem die Lackierung noch vorhanden ist sowie zwei Bereiche 54,56, in denen die Lackierung bei der Vorverarbeitung des Schrottfragments 6, beispielsweise bei einem Zerkleinerungs- oder Reinigungsschritt, entfernt wurde, so dass die darunter liegende Metalloberfläche freiliegt.

Die Auswerteeinrichtung 48 analysiert das Bild 50 mit bildverarbeitungstechnischen Methoden, um die verunreinigungsfreien Bereiche 54, 56 zu identifizieren. Hierzu kann beispielsweise ausgenutzt werden, dass die freiliegende Metalloberfläche eine andere Farbe oder ein anderes Reflexionsvermögen aufweist als eine Lackschicht oder eine anderweitig verunreinigte Oberfläche.

Insbesondere kann die Auswerteeinrichtung 48 zur Analyse des Bilds 50 zunächst durch Vergleich der Farbwerte benachbarter Bildpunkte verschiedene Bereiche in den Bilddaten des Bilds 50 bestimmen. Insbesondere kann die Auswerteeinrichtung 48 die äußere (im vorliegenden Beispiel fünfeckige) Außenkontur des Schrottfragments 6' bestimmen, die das Schrottfragment 6' vom Bereich des Hintergrunds (z.B. einem Transportband, auf dem das Schrottfragment 6' transportiert wird) trennt. Innerhalb des Schrottfragments 6' können von der Auswerteeinrichtung 48 zudem der lackierte Bereich 52 und die verunreinigungsfreien Bereiche 54, 56 identifiziert werden. Durch Vergleich der durchschnittlichen Farbwerte der Bereiche 52, 54 und 56 und ggf. des Hintergrundbereichs mit einem an einem sauberen Aluminiumfragment bestimmten Referenzwert kann die Auswerteeinrichtung 48 sodann feststellen, dass es sich bei den Bereichen 54 und 56 um verunreinigungsfreie Bereiche handelt.

Die Steuerungseinrichtung 14 ist so konfiguriert, dass sie für die Analyse des Schrottfragments 6' eine Messposition 20' in einem von der Auswerteeinrichtung 48 bestimmten verunreinigungsfreien Bereich 54, 56 auswählt und die Positionierungseinrichtung 24 der Analyseeinrichtung 8 so ansteuert, dass der Laserstrahl 18 für die Messung am Schrottfragment 6' auf die ausgewählte Messposition 20 gerichtet wird. Die ausgewählte Messposition 20' ist in Fig. 3 exemplarisch in dem verunreinigungsfreien Bereich 52 eingezeichnet. Durch diese Auswahl der Messposition 20' wird erreicht, dass die lokale Messung an einer möglichst sauberen Stelle auf der Oberfläche 22 des Schrottfragments 6' erfolgt, so dass die Zusammensetzungsinformation 40 die Legierungszusammensetzung des Schrottfragments 6 möglichst zuverlässig wiedergibt.

Die Zusammensetzungsinformation 40 kann beispielsweise genutzt werden, um die Legierungszusammensetzung einer Menge Schrottfragmente 6 zu ermitteln. Zu diesem Zweck können die vereinzelten Schrottfragmete 6 nacheinander mit der Analyseeinrichtung 8 analysiert und aus den resultierenden einzelnen Zusammensetzungsinformationen 40 die mittlere Legierungszusammensetzung der Menge Schrottfragmente 6 bestimmt werden.

Weiterhin können die Zusammensetzungsinformationen 40 auch zur Sortierung der Schrottfragmente 6 verwendet werden, indem die optionale Sortiereinrichtung 12 von der Steuerungseinrichtung 14 abhängig von der jeweils für die einzelnen Schrottfragmente 6 bestimmten Zusammensetzungsinformation 40 gesteuert wird.

Die Sortiereinrichtung 12 ist in Fig. 1 exemplarisch als ansteuerbare, zwischen einer geschlossenen Stellung (durchgezogene Linie) und einer geöffneten Stellung (gestrichelte Linie) bewegbare Klappe 58 dargestellt. Die Klappe 58 kann durch die Sortiereinrichtung 12 beispielsweise so angesteuert werden, dass sich die Klappe 58 in die geöffnete Stellung bewegt, wenn der Gehalt einer Legierungskomponente, z.B. Mg, aus der Zusammensetzungsinformation 40 oberhalb eines vorgegebenen Grenzwertes liegt, und dass sich die Klappe 58 in die geschlossene Stellung bewegt, wenn der Gehalt der Legierungskomponente unterhalb des vorgegebenen Grenzwertes liegt. Damit lassen sich die Schrottfragmente abhängig von ihrer mit der Analyseeinrichtung 8 bestimmten Legierungszusammensetzung legierungsspezifisch sortieren.

Die ansteuerbare Klappe 58 ist nur ein einfaches Beispiel für die Sortiereinrichtung 12. Stattdessen können auch andere Einrichtungen zum Sortieren der Schrottfragmente 6 vorgesehen sein. Beispielsweise können die einzelnen Schrottfragmente auch pneumatisch sortiert werden, indem die Schrottfragmente durch einen kurzen, starken Luftstoß aussortiert werden, wenn sie ein bestimmtes Kriterium bezüglich ihrer Zusammensetzung erfüllen.

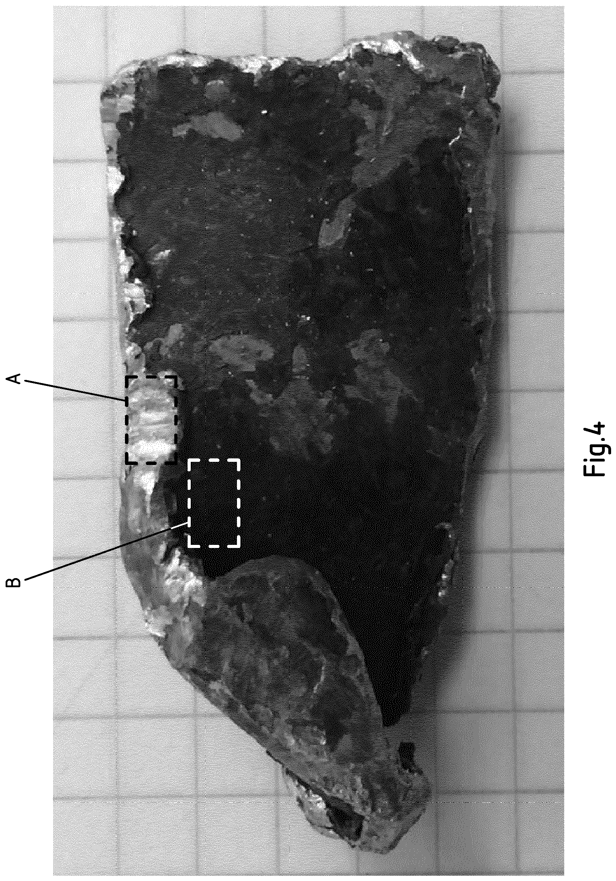

Die Auswertung der Bilddaten, wie z.B. der Bilddaten des Bilds 50 aus Fig. 3, wird im Folgenden anhand eines konkreten Beispiels dargestellt. Hierzu zeigt Fig. 4 Bilddaten eines Bildes, das mit der Kamera 46 von der Oberfläche eines Schrottfragments aufgenommen wurde. Bei dem betreffenden Schrottfragment handelt es sich um ein Aluminiumschrottfragment, das bereichsweise eine dunkle Lackierung aufweist und bei dem das Aluminium in anderen Bereichen freiliegt.

Die Auswerteeinrichtung 48 kann die in Fig. 4 dargestellten Bilddaten beispielsweise wie folgt verarbeiten:
Zunächst bestimmt die Auswerteeinrichtung 48 durch bildverarbeitungstechnische Methoden (z.B. durch Kantendetektion) die Umrisse des Schrottfragments auf der Unterlage, um den Bildbereich des Schrottfragments vom Bildbereich des Transportbands 10 zu unterscheiden.

Anschließend wählt die Auswerteeinrichtung 48 innerhalb des Bildbereichs des Schrottfragments mehrere Bereiche aus, um anhand der Farb- und/oder Helligkeitswerte in jedem Bereich zu bestimmen, ob es sich um einen verunreinigten oder verunreinigungsarmen bzw. -freien Bereich handelt. Fig. 4 zeigt beispielhaft zwei Bereiche A und B, in denen von der Auswerteeinrichtung 48 eine solche Analyse durchgeführt wird. Der Bereich A zeigt einen verunreinigungsarmen und der Bereich B einen lackierten und somit verunreinigten Bereich der Oberfläche des Schrottfragments.

Zur Bestimmung, ob es sich bei den Bereichen A und B jeweils um einen verunreinigten oder verunreinigungsarmen bzw. -freien Bereich handelt, bestimmt die Auswerteeinrichtung 48 im vorliegenden Beispiel die Verteilung der Werte für die einzelnen Farbkomponenten der Pixel im jeweiligen Bereich, beispielsweise für die Farbkomponenten R, G und B des RGB-Farbraums. Alternativ zum RGB-Farbraum kann die Auswertung auch in einem anderen Farbraum erfolgen, beispielsweise dem YCbCr-Farbraum.

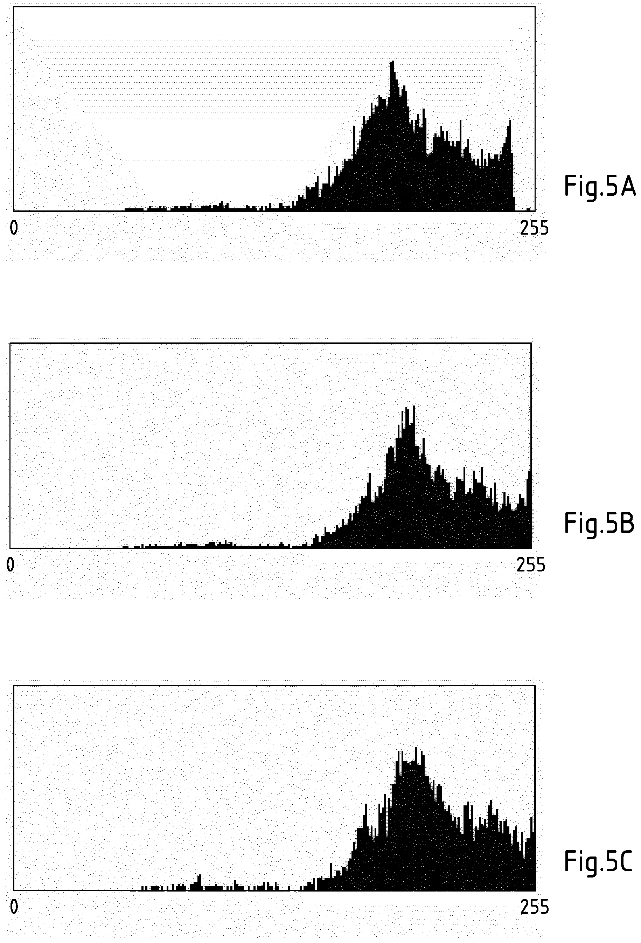

In Fig. 5a-c sind die Ergebnisse einer solchen Auswertung für den Bereich A in Form von Histogrammen dargestellt. Fig. 5a zeigt ein Histogramm für die R-Komponente (Rot). Auf der Abszisse sind von links nach rechts die möglichen Werte für die R-Komponente eines Pixels aufgetragen, und zwar im vorliegenden Beispiel die Werte von 0 (= kein Rotanteil) bis 255 (= maximaler Rotanteil), da die Kamera 46 Bilddaten mit einer Farbtiefe von 8 Bit pro Farbkanal R, G und B und Pixel liefert. Auf der Ordinate ist für jeden Wert auf der Abszisse zwischen 0 und 255 jeweils die Anzahl der Pixel aufgetragen, die im Bereich A einen entsprechenden Wert für die R-Komponente aufweisen. Fig. 5b und 5c zeigen entsprechende Histogramme für die G(Grün)-Komponente (Fig. 5b) und die B(Blau)-Komponente (Fig. 5c).

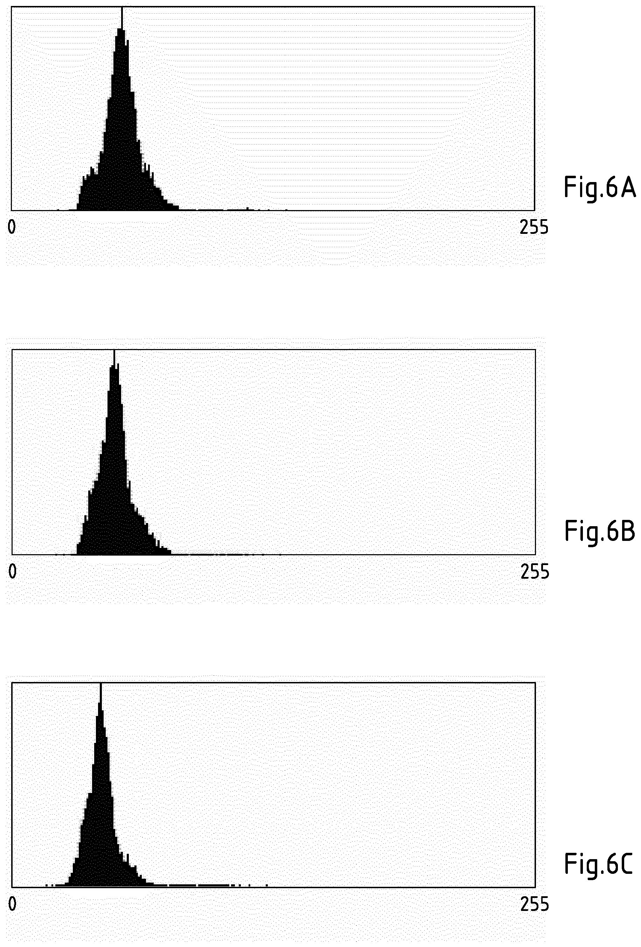

In Fig. 6a-c sind analog zu Fig. 5a-c die Ergebnisse einer Auswertung für den Bereich B dargestellt. Es zeigen Fig. 6a das Histogramm für die R-Komponente, Fig. 6b das Histogramm für die G-Komponente und Fig. 6c das Histogramm für die B-Komponente.

Der relative Vergleich der Histogramme aus dem Bereich A mit den Histogrammen aus dem Bereich B zeigt, dass die Pixel im Bereich A im Durchschnitt höhere Werte aufweisen, d.h. heller sind, als die Pixel im Bereich B. Weiterhin zeigt ein Vergleich der Histogramme, dass die Werte der Pixel im Bereich A eine breitere Streuung aufweisen als im Bereich B. Die Auswerteeinrichtung 48 kann dieses Ergebnis beispielsweise dadurch ableiten, dass sie die Mittelwerte bzw. Varianzen der Histogramme für die einzelnen Farbkanäle und Bereiche bestimmt und die Ergebnisse für den Bereich A mit den entsprechenden Ergebnissen für den Bereich B vergleicht.

Auf Basis dieser Auswertung kann die Auswerteeinrichtung 48 darauf schließen, dass der Bereich A eine geringere Verunreinigung aufweist als der Bereich B. Die Auswerteeinrichtung 48 kann auf diese Weise auf Basis der zuvor beschriebenen Auswertung den Bereich A als verunreinigungsarmen Bereich identifizieren.

Anstelle eines relativen Vergleichs zwischen verschiedenen Bildbereichen eines Schrottfragments kann die Auswerteeinrichtung 48 auch einen relativen Vergleich zwischen einem Bildbereich des Schrottfragments und Referenzwerten von einer Referenzprobe durchführen, um zu bestimmen, ob es sich bei einem Bereich des Schrottfragments um einen verunreinigten Bereich oder um einen verunreinigungsarmen bzw. -freien Bereich handelt.

Zu diesem Zweck können der Vorrichtung 2 vor der Sortierung einer Schrottmenge einzelne Schrottfragmente aus der Schrottmenge als Referenzproben zugeführt und mit der Kamera 46 entsprechende Bilddaten aufgenommen werden. In den Bilddaten können dann beispielsweise durch einen Benutzer über eine dafür vorgesehene Eingabeeinrichtung Bereiche als verunreinigte Bereiche (analog zum Bereich B aus Fig. 4) bzw. verunreinigungsarme bzw. -freie Bereiche (analog zum Bereich A aus Fig. 4) markiert werden. Die Auswerteeinrichtung 48 kann dann die Bilddaten der Referenzproben in den markierten Bereichen auswerten, beispielsweise als Histogramm analog zu Fig. 5a-c bzw. Fig. 6a-c, und entsprechende Referenzwerte für verunreinigungsarme/-freie sowie verunreinigte Bereiche ableiten. Als Referenzwert kommen z.B. wiederum der Mittelwert und/oder die Varianz der Farbwertverteilungen (Histogramme) in Frage.

Anschließend können der Vorrichtung 2 die restlichen Schrottfragmente aus der Schrottmenge zugeführt werden. Die mit der Kamera 46 erfassten Bilddaten für die einzelnen Schrottfragmente können dann wie oben für Fig. 4 beschrieben ausgewertet werden, wobei die Ergebnisse für einen ausgewählten Bereich mit den zuvor bestimmten Referenzwerten verglichen werden, um einen verunreinigungsarmen bzw. -freien Bereich zu bestimmen.

Auf diese Weise kann die Analyseeinrichtung 8 der Vorrichtung 2 die Messposition für die lokale Messung dann in dem von der Erfassungseinrichtung 44 bestimmten, verunreinigungsarmen bzw. -freien Bereich positionieren.

## Patentansprüche

1. Vorrichtung (2) zur Legierungsanalyse von Schrottfragmenten (6, 6') aus Metall,
- mit einer Analyseeinrichtung (8), die dazu eingerichtet ist, mittels einer lokalen Messung an einer Messposition (20, 20') auf der Oberfläche (22) eines zu analysierenden Schrottfragments (6,6') eine Zusammensetzungsinformation (40) über die Legierungszusammensetzung des Schrottfragments (6, 6') zu bestimmen,
- wobei die Vorrichtung (2) eine Erfassungseinrichtung (44) aufweist, die dazu eingerichtet ist, einen verunreinigungsarmen oder verunreinigungsfreien Bereich (54, 56) auf der Oberfläche (22) des zu analysierenden Schrottfragments (6, 6') zu bestimmen, und
- wobei die Analyseeinrichtung (8) dazu eingerichtet ist, die Messposition (20, 20') für die lokale Messung in dem von der Erfassungseinrichtung (44) bestimmten Bereich (54, 56) zu positionieren,
**dadurch gekennzeichnet, dass** die Erfassungseinrichtung (44) eine Bilderfassungseinrichtung (46) zur Aufnahme von Bilddaten (50) der Oberfläche (22) des zu analysierenden Schrottfragments (6, 6') aufweist sowie eine Auswerteeinrichtung (48), die dazu eingerichtet ist, einen verunreinigungsarmen oder verunreinigungsfreien Bereich (54, 56) auf der Oberfläche (22) des zu analysierenden Schrottfragments (6, 6') aus den von der Bilderfassungseinrichtung (46) bereitgestellten Bilddaten (50) zu bestimmen, und zwar durch Vergleich der Bilddaten (50) mit Referenzwerten oder aus den Bilddaten (50) abgeleiteter Größen mit Referenzwerten, insbesondere durch Vergleich von Farb- und/oder Helligkeitswerten aus einem Bereich (A, B) der Bilddaten (50) mit Referenzwerten.

2. Vorrichtung nach Anspruch 1,
**dadurch gekennzeichnet, dass** die Analyseeinrichtung (8) für eine spektroskopische Messung eingerichtet ist, insbesondere für eine laserinduzierte Plasmaspektroskopiemessung.

3. Vorrichtung nach Anspruch 1 oder 2,
**dadurch gekennzeichnet, dass** die Analyseeinrichtung (8) eine Positionierungseinrichtung (24) aufweist, um einen Laserstrahl (18) auf eine ausgewählte Messposition (20, 20') zu richten.

4. Vorrichtung nach einem der Ansprüche 1 bis 3, weiter umfassend eine Fördereinrichtung (10) zum Transport von Schrottfragmenten (6, 6') durch einen Analysebereich, in dem die Messung durch die Analyseeinrichtung (48) erfolgt.

5. Vorrichtung nach einem der Ansprüche 1 bis 4, weiter umfassend eine Vereinzelungseinrichtung (4), die dazu eingerichtet ist, eine Menge Schrottfragmente (6, 6') zu vereinzeln, so dass die Schrottfragmente (6, 6') der Analyseeinrichtung (8) vereinzelt zugeführt werden.

6. Vorrichtung nach einem der Ansprüche 1 bis 5, weiter umfassend eine Sortiereinrichtung, die dazu eingerichtet ist, Schrottfragmente abhängig von der jeweils bestimmten Zusammensetzungsinformation zu sortieren.

7. Vorrichtung nach einem der Ansprüche 1 bis 6, aufweisend eine Steuerungseinrichtung (14), die dazu eingerichtet ist, die Vorrichtung gemäß einem Verfahren nach einem der Patentansprüche 8 bis 12 zu steuern.

8. Verfahren zur Legierungsanalyse von Schrottfragmenten (6, 6') aus Metall, insbesondere unter Verwendung einer Vorrichtung (2) nach einem der Ansprüche 1 bis 7,
- bei dem ein verunreinigungsarmer oder verunreinigungsfreier Bereich (54, 56) auf der Oberfläche (22) eines zu analysierenden Schrottfragments (6, 6') bestimmt wird,
- bei dem eine Messposition (20, 20') in dem bestimmten Bereich (54, 56) ausgewählt wird und
- bei dem mittels einer lokalen Messung an der ausgewählten Messposition (20, 20') auf der Oberfläche (22) des Schrottfragments (6, 6') eine Zusammensetzungsinformation (40) über die Legierungszusammensetzung des Schrottfragments (6, 6') bestimmt wird,
**dadurch gekennzeichnet, dass** Bilddaten (50) von einem zu analysierenden Schrottfragment (6, 6') aufgenommen werden und der verunreinigungsarme oder verunreinigungsfreie Bereich (54, 56) auf der Oberfläche (22) des zu analysierenden Schrottfragments (6, 6') aus den Bilddaten (50) bestimmt wird, und zwar durch Vergleich der Bilddaten (50) mit Referenzwerten oder aus den Bilddaten (50) abgeleiteter Größen mit Referenzwerten, insbesondere durch Vergleich von Farb- und/oder Helligkeitswerten aus einem Bereich (A, B) der Bilddaten (50) mit Referenzwerten.

9. Verfahren nach Anspruch 8,
**dadurch gekennzeichnet, dass** zur Durchführung der Messung ein Laserstrahl (18) auf die ausgewählte Messposition (20, 20') gerichtet wird.

10. Verfahren nach Anspruch 8 oder 9,
**dadurch gekennzeichnet, dass** die lokale Messung an der ausgewählten Messposition (20, 20') auf der Oberfläche (22) des Schrottfragments (6, 6') eine spektroskopische Messung ist, insbesondere eine laserinduzierte Plasmaspektroskopiemessung.

11. Verfahren nach einem der Ansprüche 8 bis 10,
**dadurch gekennzeichnet, dass** eine bereitgestellte Menge Schrottfragmente (6, 6') vereinzelt wird und für die vereinzelten Schrottfragmente (6, 6') Zusammensetzungsinformationen (40) bestimmt werden.

12. Verfahren nach einem der Ansprüche 8 bis 11,
**dadurch gekennzeichnet, dass** Schrottfragmente (6, 6') abhängig von der jeweils bestimmten Zusammensetzungsinformation sortiert werden.

## Claims

1. Apparatus (2) for analysing the alloy composition of metal scrap fragments (6, 6'),
- having an analysing system (8) configured to determine, by means of a local measurement at a measurement position (20, 20') on the surface (22) of a scrap fragment (6, 6') to be analysed, a piece of composition information (40) about the alloy composition of the scrap fragment (6, 6'),
- wherein the apparatus (2) has a detecting system (44) configured to determine a region (54, 56) low in contamination or free of contamination on the surface (22) of the scrap fragment (6, 6') to be analysed, and
- wherein the analysing system (8) is configured to position the measurement position (20, 20') for the local measurement in the region (54, 56) determined by the detecting system (44),
**characterised in that**
the detecting system (44) has an image capturing system (46) to record image data (50) of the surface (22) of the scrap fragment (6, 6') to be analysed and an evaluating system (48) configured to determine a region (54, 56) low in contamination or free of contamination on the surface (22) of the scrap fragment (6, 6') to be analysed from the image data (50) provided by the image capturing system (46), namely by comparing the image data (50) with reference values or quantities derived from the image data (50) with reference values, in particular by comparing colour and/or brightness values from a region (A, B) of the image data (50) with reference values.

2. Apparatus according to claim 1, **characterised in that** the analysing system (8) is configured for a spectroscopic measurement, in particular for a laser-induced breakdown spectroscopy measurement.

3. Apparatus according to claim 1 or 2, **characterised in that** the analysing system (8) has a positioning system (24) for directing a laser beam (18) to a selected measurement position (20, 20').

4. Apparatus according to any one of claims 1 to 3, further comprising a conveying system (10) for transporting scrap fragments (6, 6') through an analysis region in which the measurement by the analysing system (8) takes place.

5. Apparatus according to any one of claims 1 to 4, further comprising a separating system (4) configured to separate a quantity of scrap fragments (6, 6') so that the scrap fragments (6, 6') are supplied in a separated manned to the analysing system (8).

6. Apparatus according to any one of claims 1 to 5, further comprising a sorting system configured to sort scrap fragments as a function of the piece of composition information determined in each case.

7. Apparatus according to any one of claims 1 to 6, having a controller (14) configured to control the apparatus according to a method according to any one of claims 8 to 12.

8. Method for analysing the alloy composition of metal scrap fragments (6, 6'), in particular using an apparatus (2) according to any one of claims 1 to 7,
- in which a region (54, 56) low in contamination or free of contamination is determined on the surface (22) of a scrap fragment (6, 6') to be analysed,
- in which a measurement position (20, 20') is selected in the determined region (54, 56) and
- in which a piece of composition information (40) about the alloy composition of the scrap fragment (6, 6') is determined by means of a local measurement at the selected measurement position (20, 20') on the surface (22) of the scrap fragment (6, 6'),
**characterised in that**
image data (50) of a scrap fragment (6, 6') to be analysed is recorded and the region (54, 56) low in contamination or free of contamination is determined on the surface (22) of the scrap fragment (6, 6') to be analysed from the image data (50), namely by comparing the image data (50) with reference values or variables derived from the image data (50) with reference values, in particular by comparing colour and/or brightness values from a region (A, B) of the image data (50) with reference values.

9. Method according to claim 8, **characterised in that**, in order to perform the measurement, a laser beam (18) is directed onto the selected measurement position (20, 20').

10. Method according to claim 8 or 9, **characterised in that** the local measurement at the selected measurement position (20, 20') on the surface (22) of the scrap fragment (6, 6') is a spectroscopic measurement, in particular a laser-induced breakdown spectroscopy measurement.

11. Method according to any one of claims 8 to 10, **characterised in that** a provided quantity of scrap fragments (6,6') is separated and pieces of composition information (40) is determined for the separated scrap fragments (6, 6').

12. Method according to any one of claims 8 to 11, **characterised in that** the scrap fragments (6, 6') are sorted as a function of the piece of composition information determined in each case.

## Revendications

1. Dispositif (2) pour l'analyse d'alliage de fragments de déchet (6, 6') en métal,
- comportant un équipement d'analyse (8) conçu pour déterminer, au moyen d'une mesure locale à une position de mesure (20, 20') sur la surface (22) d'un fragment de déchets (6, 6') à analyser, une information de composition (40) concernant la composition d'alliage du fragment de déchet (6, 6'),
- le dispositif (2) comportant un équipement de détection (44) conçu pour déterminer une zone (54, 56) pauvre ou exempte d'impuretés sur la surface (22) du fragment de déchet (6, 6') à analyser, et
- l'équipement d'analyse (8) étant conçu pour définir la position de mesure (20, 20') pour la mesure locale dans la zone (54, 56) déterminée par l'équipement de détection (44).
**caractérisé en ce que**
l'équipement de détection (44) présente un équipement de saisie d'image (46) pour la saisie de données d'image (50) de la surface (22) du fragment de déchet (6, 6') à analyser, ainsi qu'un équipement d'évaluation (48) qui est conçu pour déterminer une zone (54, 56) pauvre en ou exempte d'impuretés sur la surface (22) du fragment de déchet (6, 6') à analyser à partir des données d'image (50) mises à disposition par l'équipement de saisie d'image (46), ceci par comparaison des données d'image (50) avec des valeurs de référence ou des grandeurs dérivées des données d'image (50) avec des valeurs de référence, notamment par comparaison de valeurs de couleur et/ou de luminosité provenant d'une zone (A, B) des données d'image (50) avec des valeurs de référence.

2. Dispositif selon la revendication 1, **caractérisé en ce que** l'équipement d'analyse (8) est conçu pour une mesure spectroscopique, notamment pour une mesure de spectroscopie d'émission atomique de plasma induite par laser.

3. Dispositif selon la revendication 1 ou 2, **caractérisé en ce que** l'équipement d'analyse (8) présente un équipement de positionnement (24) pour diriger un faisceau laser (18) sur une position de mesure (20, 20') sélectionnée.

4. Dispositif selon l'une des revendications 1 à 3, comportant en outre un équipement de transport (10) pour le transport de fragments de déchet (6, 6') à travers une zone d'analyse dans laquelle a lieu la mesure par l'équipement d'analyse (48).

5. Dispositif selon l'une des revendications 1 à 4, comportant en outre un équipement de séparation (4) qui est conçu pour séparer une quantité de fragments de déchet (6, 6'), de sorte à amener séparément les fragments de déchet (6, 6') à l'équipement d'analyse (8).

6. Dispositif selon l'une des revendications 1 à 5, comportant en outre un équipement de triage qui est conçu pour trier des fragments de déchet en fonction de l'information de composition respectivement déterminée.

7. Dispositif selon l'une des revendications 1 à 6, présentant un équipement de commande (14) qui est conçu pour commander le dispositif conformément à un procédé selon l'une des revendications 8 à 12.

8. Procédé d'analyse d'alliage de fragments de déchet (6, 6') en métal, notamment en utilisant un dispositif (2) selon l'une des revendications 1 à 7,
- dans lequel une zone (54, 56) pauvre en ou exempte d'impuretés sur la surface (22) d'un fragment de déchets (6, 6') à analyser est déterminée,
- dans lequel une position de mesure (20, 20') est sélectionnée dans la zone (54, 56) déterminée et
- dans lequel une information de composition (40) concernant la composition d'alliage du fragment de déchets (6, 6') est déterminée à l'aide d'une mesure locale au niveau de la position de mesure (20, 20') sélectionnée sur la surface (22) du fragments de déchet (6, 6'),
**caractérisé en ce que** des données d'image (50) d'un fragment de déchet (6, 6') à analyser sont enregistrées et la zone (54, 56) pauvre en ou exempte d'impuretés sur la surface (22) du fragments de déchets (6, 6') à analyser est déterminée à partir des données d'image (50), ceci par comparaison des données d'image (50) avec des valeurs de référence ou à partir de grandeurs dérivées des données d'image (50) avec des valeurs de référence, notamment par comparaison de valeurs de couleur et/ou de luminosité provenant d'une zone (A, B) des données d'image (50) avec des valeurs de référence.

9. Procédé selon la revendication 8, **caractérisé en ce que**, pour l'exécution de la mesure, un faisceau laser (18) est dirigé sur la position de mesure (20, 20') sélectionnée.

10. Procédé selon la revendication 8 ou 9, **caractérisé en ce que** la mesure locale à la position de mesure (20, 20') sélectionnée sur la surface (22) du fragment de déchet (6, 6') est une mesure spectroscopique, notamment une mesure de spectroscopie d'émission atomique de plasma induite par laser.

11. Procédé selon l'une des revendications 8 à 10, **caractérisé en ce qu'**une quantité de fragments de déchet (6, 6') mise à disposition est séparée et des informations de composition (40) sont déterminées pour les fragments de déchets (6, 6') séparés.

12. Procédé selon l'une des revendications 8 à 11, **caractérisé en ce que** les fragments de déchet (6, 6') sont triés en fonction de l'information de composition respectivement déterminée.
